# EUROPEAN PATENT APPLICATION

(11) **EP 2 482 215 A2**
(43) Date of publication of application: **01.08.2012**
(21) Application number: 11177651.4
(22) Date of filing: 16.08.2011
(51) Int. Cl.: G06F 19/00

(54) **Integrated managing system for human body conditions and managing method for the same**

(30) Priority: 31.01.2011 TW 100103663
(71) Applicant: Yuekang HealthCare Management Consultans, Inc., Yongkang Dist., Tainan City 710 Chinese Taipei (CN)
(72) Inventor: Chen, Jiunn-Rong, 710 Yongkang Dist., Tainan City (TW); Liao, Shiu-Min, 710 Yongkang Dist., Tainan City (TW); Juan, Chun-Lin, 710 Yongkang Dist., Tainan City (TW)
(74) Representative: 2K Patentanwälte Blasberg Kewitz & Reichel

(57) **Abstract**

An integrated managing system (1) includes a server (2) and a database (3), the server (2) establishes a website (21) thereon and the database (3) stores disease data (311) and personal health records (PHR, 312) for at least one care-receiver therein. The website (21) includes a data source platform (24), an integrating and predicting platform (25), a health care module (26), and an event managing module (27). The data source platform (24) receives, saves and manages data of at least one care-receiver. The integrating and predicting platform (25) integrates established diseases information of each care-receiver, and predicts probably developing diseases risk of each care-receiver. The health care module (26) generates individual care plans according to the established diseases and the probably developing diseases of each care-receiver. The event managing module (27) notifies a caregiver to manage an upcoming follow-up event when the event is scheduled for the care-receiver.

## Description

### BACKGROUND OF THE INVENTION

### 1. Field of the invention

The invention relates to a managing system, and in particularly to a managing system which can be used to manage human body conditions.

### 2. Description of Prior Art

Traditionally, when a patient goes to a hospital or a clinic, the diagnosis of the patient will be written on chart paper after medical procedures such as interviewing, laboratory testing and medical imaging. Then, the chart paper will be arranged in accordance with the order of recorded date, so as to make a specific medical record of the patient.

However, paper medical records need large storage space to store, and paper medical records have disadvantage for doctors to inquire, not to mention the possible breakage and loss of the paper medical records, If medical records of patients are incomplete, it may lead to wrong judgment or misdiagnosis when the patients come back to the hospital later on.

As mentioned above, there are growing numbers of hospitals and clinics using a healthcare information system (HIS) to manage electronic medical records of patients to avoid those mentioned disadvantages. HIS is an electronic managing system for recording electronic medical records. HIS receives the electronic medical records of patients after medical procedures, such as interviewing, laboratory testing and medical imaging via a computer. HIS classifies those inputted electronic medical records into different categories, and then saves those electronic medical records to a database respectively according to the order of executing date of those medical procedures.

It has advantage that, once a patient was interviewed, laboratory tested or medical imaged, the doctor can easily input personal identifying information of the patient into the computer to enquire the electronic medical records of the patient very quickly when the patient comes back to the hospital again.

However, HIS are for the purpose of doctor's documentation and easy retrieval, which is not easily accessed by the patients. Usually patients should submit additional application to inquire their own electronic medical records, which is very inconvenient. Moreover, patients should take notes by themselves, such as the time of taking medicine or the time of scheduled visit. The patients can not receive any tips of reminder from the HIS.

In particularly, doctors are too busy to supervise patients to take medicine, to rehabilitate regularly, to come back to the hospital on schedule, and so on. Therefore, even if doctors make specific plans for the patients, the treatment ejects will be hampered if patients did not follow these plans.

Moreover, when the patients come to the hospital or the clinic, the doctor can only treat the patients according to the previous electronic medical record stored by HIS and current disease status. There is no system or method capable of predicting probably developing diseases that the patients may suffer in the future according to physiological data (for example, blood pressure and blood sugar) and/or non-physiological data (for example, living habits and family history). In other words, the present medical systems are just the systems for treating established diseases, not for preventing probably developing diseases.

As mentioned above, a brand new managing system is needed that can integrate data of patients to provide care service for both established diseases and probably developing diseases simultaneously. By informing a caregiver to react when the status of the patients has deteriorated, a managing system will further enhance the effectiveness of management.

### SUMMARY OF THE INVENTION

The invention is to provide an integrated managing system and managing method for human body conditions. Disease data and personal health records of care-receivers are stored by the integrated managing system, and individualized care plans will be generated for each of the care-receivers after data integration and disease prediction.

For another purpose, the invention is to provide an integrated managing system and managing method for human body conditions. When a specific event is scheduled by the integrated managing system, a caregiver will be informed automatically to manage the upcoming follow-up events for care-receivers.

For the purposes mentioned above, an integrated managing system including a server and a database is presented. The server establishes a website thereon and the database stores disease data and personal health records (PHR) for at least one care-receiver therein.

The website includes a data source platform, an integrating and predicting platform, a health care module, and an event managing module. The data source platform receives, saves and manages data of at least one care-receiver. The integrating and predicting platform integrates established diseases information of each care-receiver which relates to laboratory testing, medical imaging, drug, nutrition, exercise, personal preference, and so on, and the integrating and predicting platform also predicts probably developing diseases risk of each care-receiver. The health care module generates individualized care plans according to the established diseases and the probably developing diseases of each care-receiver. The event managing module notifies a caregiver to manage an upcoming follow-up event when the event is scheduled for the care-receiver.

In comparison with the prior art, the present invention has advantage as follows:
1. The integrated managing system can not only retrieve case data uploaded or inputted by the care-receiver, but also can retrieve medical records of the care-receiver from hospital or clinic servers. Therefore, the present invention can make the integrated data more comprehensive.
2. The integrated managing system can highly integrate established diseases information of the care-receiver to be managed and inquired easily, and also can predict probably developing diseases risk in the future that some steps can be done in advance for prevention or monitoring.
3. The integrated managing system can generate individualized care plans according to integration of the established diseases, the probably developing diseases of the care-receiver and related information, so as to treat and follow-up the established diseases regularly, effectively and to reduce the incidence of the probably developing discases according to those care plans.
4. The integrated managing system can schedule specific events to inform the caregiver to manage the upcoming follow-up events for the care-receiver when the care-receiver did not follow a preset measuring frequency to perform a scheduled physiological measure, or the performed physiological measure is regarded as abnormal, or there is a scheduled event needed to notify the care-receiver.

### BRIEF DESCRIPTION OF THE DRAWINGS

Figure 1 is a schematic view of a system of a preferred embodiment according to the present invention.
Figure 2 is a schematic view of a database of a preferred embodiment according to the present invention.
Figure 3 is a schematic view of a website of a preferred embodiment according to the present invention.
Figure 4a is a schematic view of a nutrition and exercise record managing module of a preferred embodiment according to the present invention.
Figure 4b is a schematic view of a nutrition and exercise prescription module of a preferred embodiment according to the present invention.
Figure 4c is a schematic view of a health care module of a preferred embodiment according to the present invention.
Figure 4d is a schematic view of an event managing module of a preferred embodiment according to the present invention.
Figure 4e is a schematic view of a performance evaluating module of a preferred embodiment according to the present invention.
Figure 5 is a flowchart of a preferred embodiment according to the present invention.

### DETAILED DESCRIPTION OF THE INVENTION

In cooperation with the attached drawings, the technical contents and detailed description of the present invention are described thereinafter according to a preferable embodiment, being not used to limit its executing scope. Any equivalent variation and modification made according to appended claims is all covered by the claims claimed by the present invention.

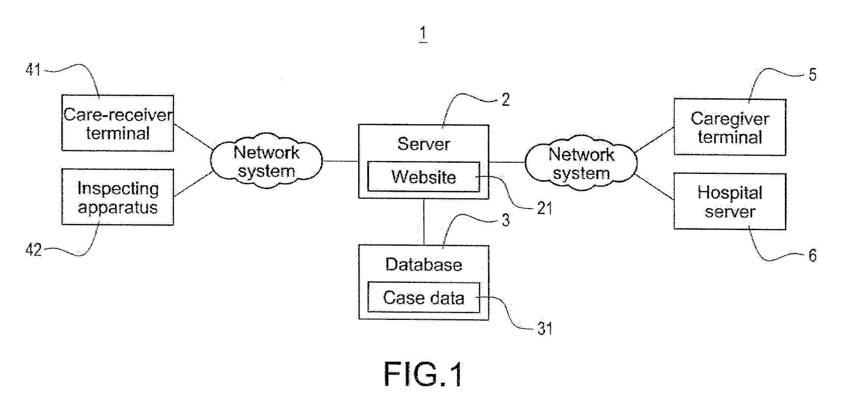

Fig. 1 is a schematic view of a system of a preferred embodiment according to the present invention. The integrated managing system 1 in the present invention mainly comprises a server 2 and a database 3 connected to the server 2, wherein the server 2 establishes a website 21 thereon, and the database 3 stores at least one case data 31 therein and each of the case data 31 is corresponding to a designated care-receiver.

The care-receiver can use a care-receiver terminal 41, such as a computer, to access to the website 21 through network system (such as internet or intranet) to inquire or input the corresponding case data 31.

The integrated managing system 1 can further comprise a measuring tool 42 which connects to the server 2 through the network system. The measuring tool 42 is set in the care-receiver's home or carried by the care-receiver. Therefore, the care-receiver can measure self physiological status via the measuring tool 42 and upload physiological measurement data to the server 2 to update the corresponding case data 31 in the database 3.

In particularly, the measuring tool 42 is used to perform the physiological measurement data, such as systolic blood pressure (SBP), diastolic blood pressure (DBP), mean arterial pressure (MAP), pulse pressure, heart rate (HR), blood sugar (BS), body temperature (BT), oxygen saturation (SPO2) and so on, but the above examples are not intended to limit the scope of the present invention.

A caregiver can use a caregiver terminal 5, such as a computer, to access to the website 21 through the network system to manage the case data 31 of the care-receiver.

The integrated managing system 1 can further comprise at least one hospital server 6 of a hospital or a clinic which connects to the server 2 through the network system. Therefore, the hospital server 6 can upload medical records of the care-receiver to the database 3 to update the case data 31 after the care-receiver completes medical procedures, such as interviewing, laboratory testing and medical imaging.

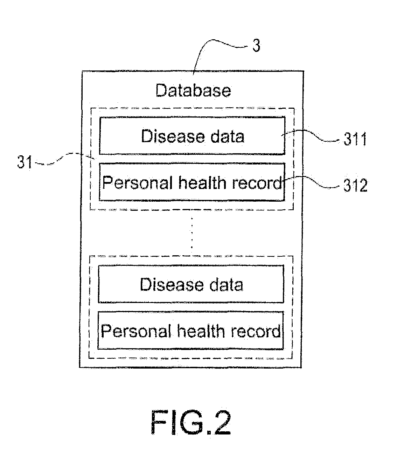

Figure 2 is a schematic view of a database of a preferred embodiment according to the present invention. The database 3 stores at least one case data 31, wherein each of the case data 31 is corresponding to one designated care-receiver and comprises a disease data 311 and a personal health record (PHR) 312 according to the care-receiver.

The disease data 311 can be the data related to diseases of the care-receiver directly, such as data of diseases, medicines, laboratory testing and medical imaging items. The PHR 312 can be the data not directly related to diseases of the care-receiver, such as data of living habits and family history of the care-receiver.

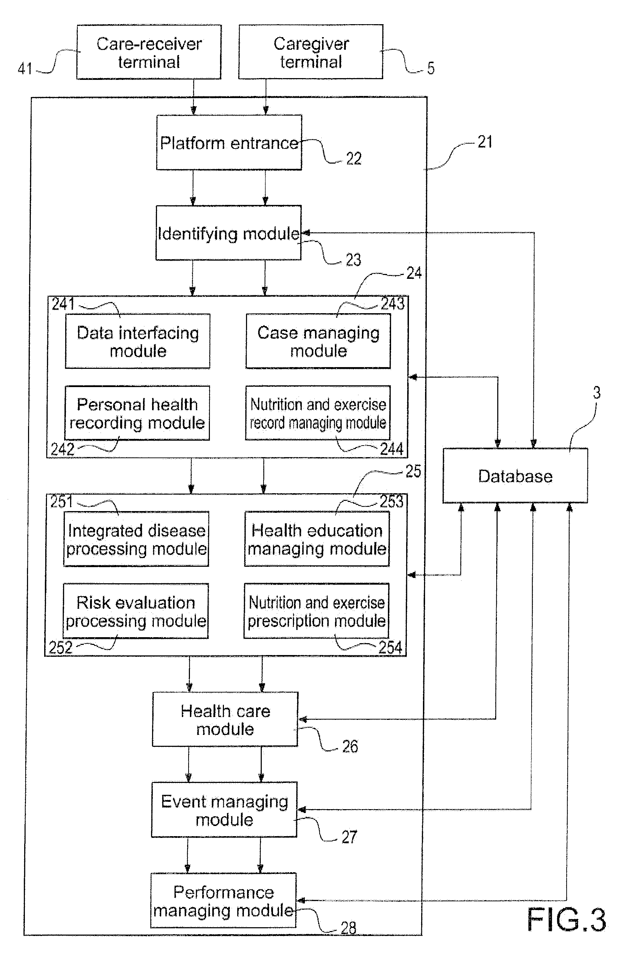

Figure 3 is a schematic view of a website of a preferred embodiment according to the present invention. The website 21 mainly comprises a platform entrance 22, an identifying module 23, a data source platform 24, an integrating and predicting platform 25, a health care module 26 and an event managing module 27.

The platform entrance 22 receives access of the care-receiver or the caregiver to login into the website 21 via an external terminal (the caregiver terminal 5 or the care-receiver terminal 41). The identifying module 23 connects to the platform entrance 22 and determines whether the accessed terminal is permitted to login into the website 21 or not. The caregiver or the care-receiver can access to the website 21 to further inquire data stored in the database 3 if he or she has passed the identification.

The data source platform 24 connects to the identifying module 23 and the data source platform 24 receives the latest case data 31 of the care-receiver to update to the database 3. The integrating and predicting platform 25 connects to the data source platform 24, the integrating and predicting platform 25 integrates established diseases information and predicts probably developing diseases risk of the care-receiver according to the case data 31 of the care-receiver.

The health care module 26 connects to the integrating and predicting platform 25. The health care module 26 generates a treatment plan according to the established diseases of the care-receiver, and also generates a preventing plan according to the probably developing diseases of the care-receiver. The event managing module 27 connects to the health care module 26, which not only generates plan related regular events, but also generates incident related events when status of the care-receiver is regarded as abnormal. Therefore, the caregiver can manage the care-receiver based on the degree of urgency of the generated events.

For another example, the event managing module 27 can also generate an event when a scheduled event needs to notify the care-receiver (for example, notify the care-receiver before executing date of a working event of the treatment plan or the preventing plan mentioned above). Therefore, the caregiver can be informed to notify the care-receiver via the generated event.

The data source platform 24 connects to the database 3 and mainly comprises a data interfacing module 241, a personal health recording module 242, a basic data managing module 243 and a nutrition and exercise record managing module 244.

The data interfacing module 241 connects to at least one hospital server 6 externally, and the data interfacing module 241 mainly receives medical records of the care-receiver from at least one hospital server 6 to store the medical records to the database 3, thus updating the case data 31.

The personal health recording module 242 receives the physiological measurement data measured and uploaded by the care-receiver via the measuring tool 42, and the personal health recording module 242 stores the physiological measurement data to the database 3 to update the disease data 311 of the case data 31.

Moreover, the personal health recording module 242 further receives data inputted manually by the care-receiver after the care-receiver accessed to the website 21, and the personal health recording module 242 then stores the received manually input data to the database 3 to update the PHR 312 of the case data 31. In particularly, the manually input data can be, for example, the data of living habits, family history, disability, questionnaire response, and so on; but those examples are not intended to limit the scope of the present invention.

The basic data managing module 243 provides manage function for the caregiver. In particularly, the basic data managing module 243 enables the caregiver to manage the case data 31 of the care-receiver according to access authority of the caregiver after the caregiver accesses to the website 21.

The nutrition and exercise record managing module 244 records diet and exercise habits of the care-receiver. In particularly, records of the habits can be filled by the care-receiver after the care-receiver accesses to the website 21, or the records can be filled by the caregiver, else, the records can also be updated automatically by an apparatus used by the care-receiver, such as a treadmill, an electronic food scale, and so on, during usage of the care-receiver.

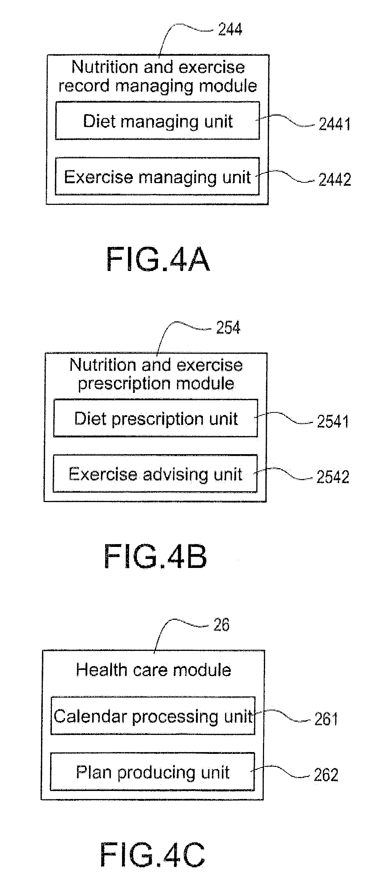

Figure 4a is a schematic view of a nutrition and exercise record managing module of a preferred embodiment according to the present invention. The nutrition and exercise record managing module 244 further comprises a diet managing unit 2441 and an exercise managing unit 2442. The diet managing unit 2441 records daily diet items of the care-receiver to calculate nutrition data daily assimilated by the care-receiver. The exercise managing unit 2442 records daily exercise items of the care-receiver to calculate calorie data consumed by the care-receiver.

According to the integrated disease data 311 and the nutrition data and the calorie data calculated by the nutrition and exercise record managing module 244, the integrated managing system 1 produces a diet item prescription and an exercise item advice to send (for example, via SMS or e-mail) to the care-receiver.

The integrating and predicting platform 25 connects to the database 3 and comprises an integrated disease processing module 251, a risk evaluation processing module 252, a health education managing module 253, and a nutrition and exercise prescription module 254.

The integrated disease processing module 251 retrieves the disease data 311 of the care-receiver. Therefore, the integrated disease processing module 251 can integrate established diseases information of the care-receiver. The risk evaluation processing module 252 retrieves the case data 31 of the care-receiver, and applies the case data 31 to a risk evaluation reference to calculate a risk evaluation value. Therefore, the risk evaluation processing module 252 can then predict probably developing diseases risk of the care-receiver by reference to the calculated risk evaluation value.

The integrating and predicting platform 25 can further retrieves the integrated disease data 311 to produce at least one diagram. Therefore, the integrated managing system I can then display the produced diagram for the caregiver and the care-receiver through the integrating and predicting platform 25. In particularly, the diagram can be, for example, a physiological data trend diagram shows the physiological measurement data of the care-receiver, a laboratory testing item trend diagram shows laboratory testing items, an medical imaging report diagram shows medical imaging results, and a medicine record diagram shows medicine records, which can be displayed in according to different time intervals but the above examples are not intended to limit the scope of the present invention.

The health education managing module 253 classifies each care-receiver into different disease groups according to the individual established diseases and the probably developing diseases. The health education managing module 253 can then send related health education information to each care-receiver separately by reference to the disease groups.

Figure 4b is a schematic view of a nutrition and exercise prescription module of a preferred embodiment according to the present invention. The nutrition and exercise prescription module 254 mainly sends the diet item prescription and the exercise item advice to the care-receiver. As shown in Fig. 4b, the nutrition and exercise prescription module 254 further comprises a nutrition prescription unit 2541 and an exercise advice unit 2542.

The nutrition prescription unit 2541 produces the diet item prescription according to the case data 31 of the care-receiver, and sends the produced diet item prescription to the care-receiver. The exercise advice unit 2542 produces the exercise advice according to the case data 31 of the care-receiver, and sends the produced exercise advice to the care-receiver. Moreover, the nutrition and exercise prescription module 254 mainly sends the diet item prescription and the exercise advice to the care-receiver through SMS or e-mail, but the above sending ways are not intended to limit the scope of the present invention.

Figure 4c is a schematic view of a health care module of a preferred embodiment according to the present invention. The health care module 26 connects to the database 3 and comprises a calendar processing unit 261 and a plan generating unit 262.

The calendar processing unit 261 provides a calendar, and receives at least one established disease or probably developing disease selected by the caregiver, and also receives a starting time set by the caregiver. When the established disease and the starting time is set, the plan generating unit 262 then applies the selected established disease to a corresponding care reference to generate the treatment plan for a certain period from the starting time. In the other hand, when the probably developing disease and the starting time is set, the plan generating unit 262 applies the selected probably developing disease to a corresponding care reference to generate the preventing plan in the same way as generating the treatment plans. Moreover, the treatment plan or the preventing plan is then displayed on the calendar in accordance with the order of current date.

For example, if the selected established disease is diabetes, the care reference according to diabetes includes "measuring blood sugar everyday", "exercising three times a week", and "clinic visit and blood test every three months", and the starting time is January 1. As mentioned above, the calendar will thereon display "blood sugar measuring" every single day, display "exercise" three times a week, and display "clinic visit and blood testing" every three month from January 1. Therefore, the care-receiver can be informed to treat diseases or to take preventive measures following the plan on the calendar.

It should be mentioned that the care-receiver can book the specific calendar mentioned above through RSS, or import the specific calendar into calendar tools which can support iCal format, such as google calendar and outlook, via iCal format.

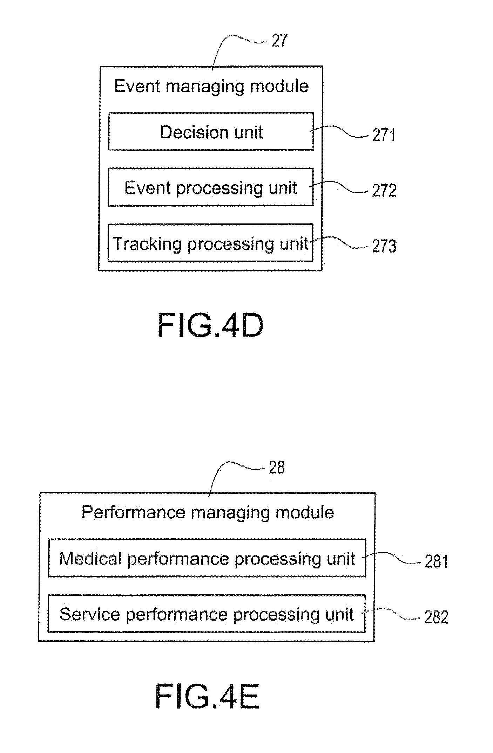

Figure 4d is a schematic view of an event managing module of a preferred embodiment according to the present invention. The event managing module 27 connects to the database 3 and further comprises a decision unit 271, an event processing unit 272 and a tracking processing unit 273.

The decision unit 271 determines whether the care-receiver follows the preset measuring frequency to measure and upload the physiological measurement data or not, and the decision unit 271 also determines whether the uploaded physiological measurement data exceeds a warning value or not. If the care-receiver did not measure and uploaded the physiological measurement data according to the preset measuring frequency, or the uploaded physiological measurement data exceeds the warning value, then the event managing module 27 generates a new event on an event list (not shown) of the caregiver via the event processing unit 272.

Therefore, the caregiver can manage the care-receiver according to the generated event on the event list, and the caregiver can further use the tracking processing unit 273 to record tracking procedures. In particularly, the event managing module 27 can also generate the event on the event list of the caregiver before the executing date of the scheduled event (for example, the working event displayed on the calendar mentioned above). Therefore, the caregiver can be informed to notify the care-receiver of the scheduled event.

It should be mentioned that the preset measuring frequency is set by the caregiver or a hospital doctor according to the diseases of the care-receiver and patient preferences. If the care-receiver did not measure the physiological measurement data according to the preset measuring frequency, or the measured physiological measurement data exceeds the warning value, the caregiver will be informed to manage according to the generated events for care-receivers.

As shown in Fig. 3, the website 21 further comprises a performance evaluating module 28. The performance evaluating module 28 connects to the database 3, and evaluates performance of care service given by the caregiver to the care-receiver. Figure 4e is a schematic view of a performance evaluating module of a preferred embodiment according to the present invention. As shown in Fig. 4e, the performance evaluating module 28 further comprises a medical performance processing unit 281 and a service performance processing unit 282.

The medical performance processing unit 281 records treatment performance of the established diseases of the care-receiver according to the care service given by the caregiver, the medical performance processing unit 281 further records disease-preventing performance of the probably developing diseases of the care-receiver according to the care service given by the caregiver. The service performance processing unit 282 records, for example, the satisfaction of the care-receiver according to the care service given by the caregiver.

In particularly, the treatment performance of the established diseases and the disease-preventing performance of the probably developing diseases can be evaluated according to the case data 31 of the care-receiver by the integrated managing system 1, and the service performance can be filled by the care-receiver after he or she accessed to the website 21, but the above examples are not intended to limit the scope of the present invention.

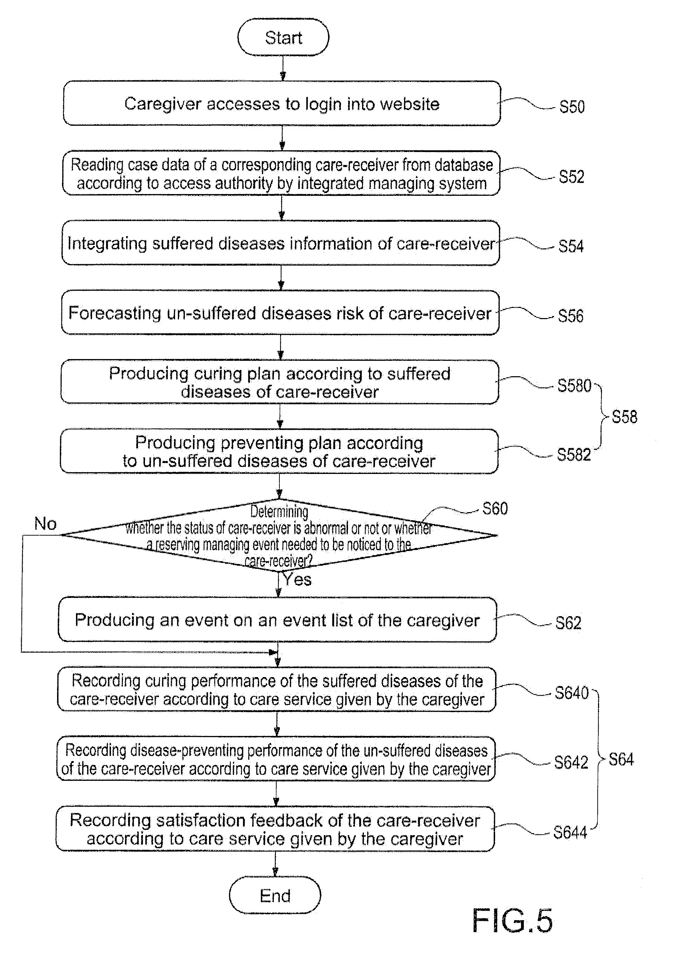

Figure 5 is a flowchart of a preferred embodiment according to the present invention. Firstly, the caregiver accesses to login into the website 21 via the caregiver terminal 5 (step S50), then the integrated managing system 1 retrieves the case data 31 of a care-receiver from the database 3 according to the access authority of the caregiver (step S52). In this embodiment, the care-receiver is one of several care targets of the caregiver.

The integrated managing system 1 then integrates the established diseases information of the care-receiver through the integrated disease processing module 251 of the integrating and predicting platform 25 (step S54), therefore, the integrated managing system 1 can produce and display those diagrams mentioned above inquired by the caregiver and the care-receiver. The integrated managing system 1 also predicts the probably developing diseases risk that the care-receiver may suffer in the future through the risk evaluation processing module 252 of the integrating and predicting platform 25 (step S56). The step S54 and the step S56 do not have the order relationship when executing, and the step S54 and the step S56 need not be performed simultaneously.

After the step S54 and/or step S56, the caregiver can then use the health care module 26 to generate at least one care plan according to the diseases of the care-receiver (step S58). In particularly, the caregiver uses the health care module 26 to generate the treatment plan according to the established diseases of the care-receiver (step S580), and the caregiver also uses the health care module 26 to generate the preventing plan according to the probably developing diseases of the care-receiver (step S582). However, the step S580 and the step S582 do not have the order relationship when executing, and the step S580 and the step S582 need not be performed simultaneously. The above example is not intended to limit the scope of the present invention.

The integrated managing system I determines whether the status of the care-receiver is abnormal or not, or whether a scheduled event needs to notify the care-receiver via the event managing module 27 (step S60). If the status of the care-receiver is regarded as abnormal, or there is a scheduled event needed to notify the care-receiver, the integrated managing system 1 generates a new event on the event list of the caregiver (step S62).

In particularly, in the step S60 as mentioned above, the status of the care-receiver is regarded as abnormal when the care-receiver did not follow the preset measuring frequency to measure or upload the physiological measurement data, or the uploaded physiological measurement data exceeds the warning value. And also in the step S60, the scheduled event usually needs to notify the care-receiver before the executing date of the working event of the treatment plan or the preventing plan. However, if there is no abnormal status and no scheduled event needed to notify in the step S60, the integrated managing system 1 need not to execute the step S62 mentioned above.

Finally, the integrated managing system 1 records the care performance of the caregiver via the performance evaluating module 28 (step S64). In particularly, the integrated managing system 1 records the treatment performance of the established diseases of the care-receiver according to the care service given by the caregiver via the medical performance processing unit 281 of the performance evaluating module 28(step S640), and the integrated managing system I also records the disease-preventing performance of the probably developing diseases of the care-receiver according to the care service given by the caregiver via the medical performance processing unit 281 (step S642).

Moreover, the integrated managing system 1 also records the satisfaction of the care-receiver according to the care service given by the caregiver via the service performance processing unit 282 of the performance evaluating module 28 (step S644). However, the step S640, the step S642, and the step S644 do not have the order relationship when executing, and the step S640, step S642, and the step S644 need not be performed simultaneously. The above example is not intended to limit the scope of the present invention.

Therefore, a supervisor of the caregiver can check the performance evaluating module 28 to realize more information such as:
1. Does the caregiver takes care of the care-receiver seriously? ;
2. Does the care-receiver receive practical help via the care service given by the caregiver? ; and
3. Does the care service given by the caregiver satisfy the care-receiver or not?

## Claims

1. An integrated managing system (1) for human body conditions comprising :
a database (3) storing at least one case data (31) therein and each of the case data being corresponding to a designated care-receiver and including a disease data (311) and a personal health record (PHR, 312) of the care-receiver; and
a server (2) connected to the database (3) and established a website (21) thereon and the website (21) including :
a data source platform (24) connected to the database (3) and receiving the case data (31) to store to the database (3);
an integrating and predicting platform (25) connected to the data source platform (24) and the database (3), the integrating and predicting platform (25) integrating established diseases information and predicting probably developing diseases risk according to the case data (31) of the care-receiver;
a health care module (26) connected to the integrating and predicting platform (25) and the database (3), the health care module (26) generating a treatment plan according to the established diseases of the care-receiver and generating a preventing plan according to the probably developing diseases of the care-receiver; and
an event managing module (27) connected to the health care module (26) and the database (3), the event managing module (27) generating an event when the status of the care-receiver is regarded as abnormal or generating the event when a scheduled event is needed to notify the care-receiver, therefore, a caregiver being informed to manage the generated event for the care-receiver.

2. The integrated managing system for human body conditions of claim 1, wherein the website (21) comprises:
a platform entrance (22) receiving access of the care-receiver or the caregiver to login into the website (21) via an external terminal (41,5); and
an identifying module (23) connected to the platform entrance (22) and identifying the accessed external terminal (41,5).

3. The integrated managing system (1) for human body conditions of claim 1 or 2, wherein the data source platform (24) comprises:
a data interfacing module (241) connected to at least one hospital server (6) externally for receiving medical records of the care-receiver from the hospital server (6) to store to the database (3) for updating the case data (31) in the database (3);
a personal health record module (242) receiving physiological measurement data from the care-receiver measured and updated via an measuring tool, storing the received physiological measurement data to the database (3) for updating the disease data (311), and the personal health record module (242) further receiving a manually inputted data inputted by the care-receiver after the care-receiver accessing to the website (21) and storing the received manually inputted data to the database (3) for updating the personal health record (312); and
a basic data managing module (243) enabling the caregiver to manage the case data (31) of the care-receiver according to access authority of the caregiver after the caregiver accessed to the website (21).

4. The integrated managing system (1) for human body conditions of claim 3, wherein the data source platform (24) further comprises a nutrition and exercise record managing module (244), the nutrition and exercise record managing module (244) recording diet and exercise habits of the care-receiver and comprising:
a diet managing unit (2441) recording daily diet items of the care-receiver to calculate nutrition data daily assimilated by the care-receiver; and
an exercise managing unit (2442) recording daily exercise items of the care-receiver to calculate calorie data daily consumed by the care-receiver;
wherein, the integrated managing system (1) integrates the disease data (311) of the care-receiver and produces a diet item prescription and an exercise item advice to send to the care-receiver according to the integrated disease data (311) and the nutrition data and the calorie data calculated by the nutrition and exercise record managing module (244).

5. The integrated managing system (1) for human body conditions of any of the preceding claims, wherein the integrating and predicting platform (25) comprises:
an integrated disease processing module (251) retrieving the disease data (311) of the care-receiver to integrate established diseases information of the care-receiver; and
a risk evaluation processing module (252) retrieving the case data (31) of the care-receiver and applying the case data (31) to a risk evaluation reference to calculate a risk evaluation value, and the risk evaluation processing module (252) predicting probably developing diseases risk of the care-receiver by reference to the calculated risk evaluation value;
wherein, the integrating and predicting platform (25) retrieves the integrated disease data (311) to produce at least one diagram to display for the caregiver and the care-receiver through the integrating and predicting platform (25).

6. The integrated managing system (1) for human body conditions of claim 5, wherein the displayed diagram selected from the group consists of physiological data trend diagram, laboratory testing item trend diagram, medical imaging report diagram and medicine record diagram.

7. The integrated managing system (1) for human body conditions of claim 5 or 6, wherein the integrating and predicting platform (25) further comprises a health education managing module (253), the health education managing module (253) classifying each care-receiver into different disease groups according to the individual established diseases information and the probably developing diseases risk and sending related health education information to each care-receiver separately by reference to the disease groups.

8. The integrated managing system (1) for human body conditions of any of claims 5 to 7, wherein the integrating and predicting platform (25) further comprises a nutrition and exercise prescription module (254), the nutrition and exercise prescription module (254) sending a diet item prescription and an exercise item advice to the care-receiver and comprising:
a nutrition prescription unit (2541) producing the diet item prescription according to the case data of the care-receiver and sending the produced diet item prescription to the care-receiver; and
an exercise advice unit (2542) producing the exercise item advice according to the case data of the care-receiver and sending the produced exercise item advice to the care-receiver.

9. The integrated managing system (1) for human body conditions of any of claim 5 to 8, wherein the health care module (26) comprises:
a calendar processing unit (261) providing a calendar, receiving at least one established disease or probably developing disease selected by the caregiver and receiving a starting time set by the caregiver; and
a plan generating unit (262) applying the selected established disease or probably developing disease to a corresponding care reference to generate the treatment plan or the preventing plan for a certain period from the starting time, and displaying the generated treatment plan or preventing plan on the calendar in accordance with an order of current date.

10. The integrated managing system (1) for human body conditions of any of claim 5 to 9, wherein the event managing module (27) further comprises:
a decision unit (271) determining whether the care-receiver follows a preset measuring frequency to measure and upload a physiological measurement data or not, said decision unit (271) further determining whether the uploaded physiological measurement data exceeds a warning value or not;
an event processing unit (272) generating the event on an event list of the caregiver when the care-receiver did not measure the physiological measurement data according to the preset measuring frequency, the updated physiological measurement data exceeded the warning value, or a scheduled event needed to notify the care-receiver; and
a tracking processing unit (273) recording tracking procedures during the caregiver managing the generated event for the care-receiver.

11. The integrated managing system (I) for human body conditions of any of the preceding claims, wherein the website (21) further comprises a performance evaluating module (28) connected to the database (3) and managing performance of care service given by the caregiver to the care-receiver and comprising:
a medical performance processing unit (281) recording treatment performance of established diseases of the care-receiver according to the care service given by the caregiver, and recording disease-preventing performance of probably developing diseases of the care-receiver according to the care service given by the caregiver; and
a service performance processing unit (282) recording satisfaction of the care-receiver according to the care service given by the caregiver.

12. An integrated managing method for human body conditions applied in an integrated managing system (1), the integrated managing system (1) comprising a server (2) and a database (3) connected to the server (2), wherein the server (3) establishes a website (21) thereon and the database (3) stores at least one case data (31) of a designated care-receiver therein and the integrated managing method for human body conditions comprising following steps of:
a) accessing to the website (21) by a caregiver;
b) retrieving the case data (31) of a corresponding care-receiver from the database (3) according to access authority of the caregiver by the integrated managing system (1);
c) integrating established diseases information of the care-receiver according to the case data (31) of the care-receiver;
d) predicting probably developing diseases risk of the care-receiver by evaluating the case data (31) of the care-receiver;
e) generating a care plan according to the established diseases and the probably developing diseases of the care-receiver;
f) determining whether status of the care-receiver is regarded as abnormal or not or whether a scheduled event needs to notify the care-receiver or not; and
g) generating a new event on an event list of the caregiver if the status of the care-receiver regarded as abnormal, or a scheduled event is needed to notify the care-receiver.

13. The integrated managing method for human body conditions of claim 12, further comprising the steps of:
h) recording treatment performance of the established diseases of the care-receiver according to care service given by the caregiver;
i) recording p disease-preventing performance of the probably developing diseases of the care-receiver according to the care service given by the caregiver; and
j) recording satisfaction of the care-receiver according to the care service given by the caregiver.

14. The integrated managing method for human body conditions of claim 12 or 13, wherein the step e) further comprises the steps of:
e1) generating a treatment plan according to the established diseases of the care-receiver; and
e2) generating a preventing plan according to the probably developing diseases of the care-receiver.

15. The integrated managing method for human body conditions of any of claims 12 to 14, wherein the status of the care-receiver is regarded as abnormal in step f) when the care-receiver did not follow a preset measuring frequency to measure and upload a physiological measurement data or when the uploaded physiological measurement data exceeded a warning value, and the scheduled event of the step t) needs to notify the care-receiver before executing date of a working event of the treatment plan or the preventing plan.
